(19) Europäisches Patentamt
European Patent Office
Office européen des brevets

(11) Veröffentlichungsnummer : **0 057 863**
**B1**

(12) # EUROPÄISCHE PATENTSCHRIFT

(45) Veröffentlichungstag der Patentschrift : **13.06.84**

(51) Int. Cl.³ : **A 61 K 31/415**, A 61 K 31/41// C07D405/06

(21) Anmeldenummer : **82100582.4**

(22) Anmeldetag : **28.01.82**

(54) **Antimikrobielle Mittel.**

(30) Priorität : **07.02.81 DE 3104380**

(43) Veröffentlichungstag der Anmeldung : **18.08.82 Patentblatt 82/33**

(45) Bekanntmachung des Hinweises auf die Patenterteilung : **13.06.84 Patentblatt 84/24**

(84) Benannte Vertragsstaaten : **BE CH DE FR GB IT LI NL SE**

(56) Entgegenhaltungen :
**EP-A- 0 060 962**
**DE-A- 2 803 870**
**DE-A- 2 943 631**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Krämer, Wolfgang, Dr.**
**Am Eckbusch 39/45**
**D-5600 Wuppertal 1 (DE)**
Erfinder : **Büchel, Karl Heinz, Prof.Dr.**
**Dabringhausener Strasse 42**
**D-5093 Burscheid (DE)**
Erfinder : **Elbe, Hans-Ludwig, Dr.**
**Dasnoeckel 59**
**D-5600 Wuppertal 11 (DE)**
Erfinder : **Kraatz, Udo, Dr.**
**Koernerstrasse 6**
**D-5090 Leverkusen (DE)**
Erfinder : **Reiser, Wolf, Dr.**
**Kiebitzweg 12a**
**D-5600 Wuppertal 1 (DE)**
Erfinder : **Regel, Erik, Ing.grad.**
**Bergerheide 72a**
**D-5600 Wuppertal 1 (DE)**
Erfinder : **Schulze, Andreas, Dr.**
**Voiswinkelerstrasse 35**
**D-5060 Bergisch-Gladbach 2 (DE)**
Erfinder : **Plempel, Manfred, Dr.**
**Pahlkestrasse 5**
**D-5600 Wuppertal 1 (DE)**

EP 0 057 863 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft die Verwendung von neuen 2-Azolylmethyl-1,3-dioxolan- und -dioxan-Derivaten als antimikrobielle Mittel, insbesondere als Antimykotika.

Es ist bereits bekannt geworden, daß 2-(2,4-Dichlorphenyl)-2-(imidazol-1-yl-methyl)-1,3-dioxolan-Derivate, wie z. B. 4-(4-Biphenylyloxymethyl)-2-(2,4-dichlorphenyl)-2-(imidazol-1-yl-methyl)-1,3-dioxolan bzw. 4-[4-(4'-Acetyl-piperazin-1-yl)-phenoxymethyl]-2-(2,4-dichlorphenyl)-2-(imidazol-1-yl-methyl)-1,3-dioxolan gute antimykotische Eigenschaften aufweisen (vergleiche DE-OS 26 02 770 bzw. DE-OS 28 04 096). Die Wirkung dieser Verbindungen ist jedoch nicht immer ganz befriedigend.

Es wurde gefunden, daß die neuen 2-Azolylmethyl-1,3-dioxolan- und -dioxan-Derivate der allgemeinen Formel

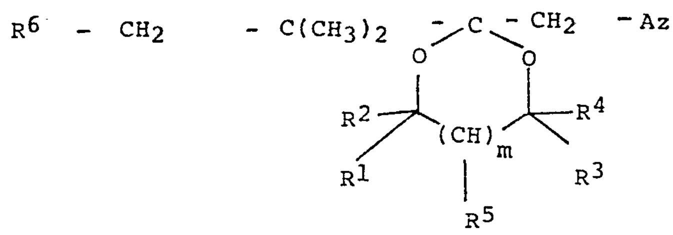

(I)

in welcher

Az für Imidazol-1-yl oder 1,2,4-Triazol-1-yl steht ;

$R^1$ für Wasserstoff oder geradkettiges und verzweigtes Alkyl mit 1 bis 4 Kohlenstoffatomen steht ;

$R^2$ für Wasserstoff oder geradkettiges und verzweigtes Alkyl mit 1 bis 4 Kohlenstoffatomen steht ;

$R^3$ für Wasserstoff oder geradkettiges und verzweigtes Alkyl mit 1 bis 4 Kohlenstoffatomen steht ;

$R^4$ für Wasserstoff, geradkettiges oder verzweigtes Alkyl mit 1 bis 4 Kohlenstoffatomen, das gegebenenfalls substituiert sein kann durch : Hydroxy, Alkoxy mit 1 bis 4 Kohlenstoffatomen, Alkylcarbonyloxy mit 1 bis 2 Kohlenstoffatomen im Alkylrest, Dialkylamino und Dialkylaminocarbonyl mit jeweils 1 bis 2 Kohlenstoffatomen in jedem Alkylteil, gegebenenfalls substituiertes Phenoxy und gegebenenfalls substituiertes Phenylalkoxy mit 1 bis 4 Kohlenstoffatomen im Alkylteil, gegebenenfalls substituiertes Phenylcarbonyloxy und gegebenenfalls substituiertes Phenylalkylcarbonyloxy mit 1 bis 4 Kohlenstoffatomen im Alkylteil, Alkylsulfonyloxy mit 1 bis 4 Kohlenstoffatomen und gegebenenfalls substituiertes Phenylsulfonyloxy, wobei jeweils als Phenyl- oder Phenoxy-substituenten genannt seien : Halogen, Alkyl mit 1 bis 4 Kohlenstoffatomen. Alkoxy und Alkylthio mit jeweils 1 bis 2 Kohlenstoffatomen, Halogenalkyl, Halogenalkoxy und Halogenalkylthio mit jeweils 1 bis 2 Kohlenstoff- und 1 bis 5 gleichen oder verschiedenen Halogenatomen, wie insbesondere Fluor- und Chloratomen, Dimethylamino, Acetylamino, Acetyl-methylamino, gegebenenfalls durch Methyl oder Acetyl substituiertes Piperazin ; sowie schließlich für gegebenenfalls substituiertes Phenyl und Phenylalkyl mit 1 bis 4 Kohlenstoffatomen im Alkylteil steht, wobei als Phenylsubstituenten die oben genannten infrage kommen ;

$R^1$ und $R^3$ außerdem gemeinsam für eine gegebenenfalls durch Alkyl mit 1 bis 4 Kohlenstoffatomen substituierte Tetra- oder Pentamethylenbrücke stehen ;

$R^5$ für Wasserstoff oder geradkettiges und verzweigtes Alkyl mit 1 bis 4 Kohlenstoffe steht ;

$R^6$ für Wasserstoff, Halogen, Cyano, geradkettiges oder verzweigtes Alkyl mit 1 bis 4 Kohlenstoffatomen, gegebenenfalls substituiertes Phenyl, wobei als Phenylsubstituenten die bei $R^4$ bereits genannten Phenylsubstituenten infrage kommen, sowie für die Gruppierungen $—X—R^7$, $—COOR^8$ und $—CONHR^9$ steht,

$R^7$ für geradkettiges oder verzweigtes Alkyl mit 1 bis 4 Kohlenstoffatomen, Halogenalkyl mit 1 bis 2 Kohlenstoff- und 1 bis 5 gleichen oder verschiedenen Halogenatomen, wie insbesondere Fluor- und Chloratomen, Cyano, sowie für gegebenenfalls substituiertes Phenyl und Phenylalkyl mit 1 bis 4 Kohlenstoffatomen im Alkylteil steht, wobei jeweils als Phenylsubstituenten die bei $R^4$ bereits genannten Phenylsubstituenten infrage kommen ;

$R^8$ für geradkettiges oder verzweigtes Alkyl mit 1 bis 4 Kohlenstoffatomen steht ;

$R^9$ für geradkettiges oder verzweigtes Alkyl mit 1 bis 4 Kohlenstoffatomen sowie für gegebenenfalls substituiertes Phenyl steht, wobei als Phenylsubstituenten die bei $R^4$ bereits genannten Phenylsubstituenten infrage kommen ;

m für 0 oder 1 steht ; und

X für Sauerstoff, Schwefel, die SO- oder $SO_2$-Gruppe steht, wobei die Gruppierung $R^6—CH_2—$ nicht für Methyl stehen darf, wenn m = 0, Az = 1,2,4-Triazol-1-yl und $R^4$ gegebenenfalls substituiertes Phenoxyalkyl bedeuten, sowie deren Säureadditionssalze gute antimikrobielle, insbesondere antimykotische Eigenschaften aufweisen.

Die Verbindungen der Formel (I) können gegebenenfalls in verschiedenen Stereoisomeren vorkommen ; vorzugsweise fallen sie in Form von Stereoisomerengemischen an. Der Verbindungen der Formel

(I) werden in der europäischen Patentanmeldung 0 057 864 beansprucht.

Überraschenderweise zeigen die erfindungsgemäß zu verwendenden 2-Azolylmethyl-1,3-dioxolan- und -dioxan-Derivate eine bessere antimykotische Wirkung, insbesondere gegen Microsporum- und Aspergillus-Arten, als die aus dem Stand der Technik bekannten Verbindungen 4-(4-Biphenylyloxymethyl)-2-(2,4-dichlormethyl)-2-(imidazol-1-yl-methyl)-1,3-dioxolan und 4-[4-(4'-Acetyl-piperazin-1-yl)-phenoxymethyl]-2-(2,4-dichlorphenyl)-2-(imidazol-1-yl-methyl)-1,3-dioxolan, welche chemisch und wirkungsmäßig naheliegende Verbindungen sind. Die erfindungsgemäße Verwendung der neuen Stoffe stellt somit eine Bereicherung der Pharmazie dar.

Die erfindungsgemäß zu verwendenden 2-Azolylmethyl-1,3-dioxolan- und -dioxan-Derivate sind durch die Formel (I) allgemein definiert.

Bevorzugt sind diejenigen Verbindungen der Formel (I), in denen

$R^1$, $R^2$ und $R^3$ die oben angegebene Bedeutung haben;

$R^4$ für Wasserstoff oder geradkettiges und verzweigtes Alkyl mit 1 bis 4 Kohlenstoffatomen steht, das gegebenenfalls substituiert sein kann durch: Hydroxy, Methoxy, Ethoxy, Dimethylamino, Dimethylaminocarbonyl, gegebenenfalls substituiertes Phenoxy und Benzyloxy, Methylcarbonyloxy, Ethylcarbonyloxy, gegebenenfalls substituiertes Phenylcarbonyloxy und Benzylcarbonyloxy, Methylsulfonyloxy, Ethylsulfonyloxy sowie gegebenenfalls substituiertes Phenylsulfonyloxy, wobei jeweils als Phenylsubstituenten genannt seien: Fluor, Chlor, Methyl, Ethyl, Methoxy, Methylthio, Trifluormethyl und Trifluormethylthio, Dimethylamino, Acetylamino, Acetylmethylamino, 4-Acetylpiperazin-1-yl, Nitro, Methylcarbonyloxy, Ethylcarbonyloxy sowie auch für gegebenenfalls substituiertes Phenyl und Benzyl steht, wobei als Phenyl- oder Phenoxy-substituenten die obengenannten infrage kommen;

$R^1$ und $R^3$ außerdem gemeinsam für Tetramethylenbrücke stehen;

$R^5$ für Wasserstoff, Methyl oder Ethyl steht;

$R^6$ für Wasserstoff, Fluor, Chlor, Brom, Methyl, Ethyl, Propyl, gegebenenfalls substituiertes Phenyl, wobei als Substituenten die bei $R^4$ bereits genannten Phenylsubstituenten infrage kommen, sowie für die Gruppierungen $—X—R^7$, $—COOR^8$ und $—CONHR^9$ steht,

$R^7$ für Methyl, Ethyl, Trifluormethyl, Cyano, sowie gegebenenfalls substituiertes Phenyl und Benzyl steht, wobei jeweils als Phenylsubstituenten die bei $R^4$ bereits genannten Phenylsubstituenten infrage kommen;

$R^8$ für Methyl oder Ethyl steht;

$R^9$ für Methyl, Ethyl sowie gegebenenfalls substituiertes Phenyl steht, wobei als Phenylsubstituenten die bei $R^4$ bereits genannten Phenylsubstituenten infrage kommen; und

Az, X und m die oben angegebene Bedeutung haben, wobei die Gruppierung $R^6—CH_2—$ nicht für Methyl stehen darf, wenn $m = 0$, Az = 1,2,4-Triazol-1-yl und $R^4$ gegebenenfalls substituiertes Phenoxyalkyl bedeuten.

Die erfindungsgemäß zu verwendenden Wirkstoffe sind noch nicht bekannt. Sie können jedoch gemäß einem eigenen Vorschlag hergestellt werden, indem man

a) substituierte 1,3-Dioxolan- und Dioxan-Derivate der Formel

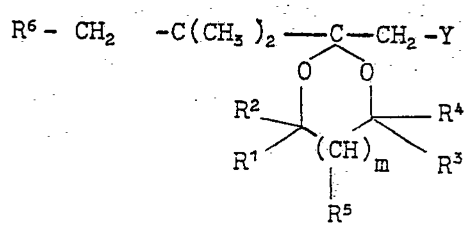

(II)

in welcher

$R^1$ bis $R^6$, und m die oben angegebene Bedeutung haben, und

Y für Halogen, insbesondere Chlor oder Brom, sowie die Gruppierung $—O—SO_2—Z$ steht, wobei

Z für Methyl oder p-Methylphenyl steht,

mit Alkalisalzen von Azolen der Formel

$$M—Az \qquad (III)$$

in welcher

Az die oben angegebene Bedeutung hat und

M für ein Alkalimetall steht,

in Gegenwart eines inerten organischen Lösungsmittels, wie beispielsweise Dimethylformamid, bei Temperaturen zwischen 60 und 150 °C umsetzt, oder

b) Azolylmethyl-keto-Derivate der Formel

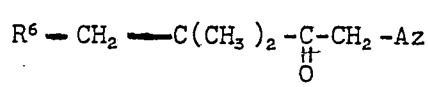

(IV)

in welcher Az und $R^6$ die oben angegebene Bedeutung haben, mit Diolen der Formel

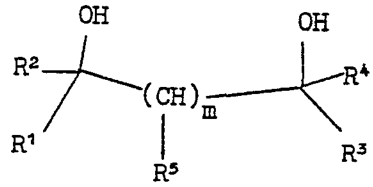

(V)

in welcher $R^1$ bis $R^5$ und m die oben angegebene Bedeutung haben, in Gegenwart eines inerten organischen Lösungsmittels, wie beispielsweise Toluol, und in Gegenwart einer starken Säure als Katalysator, wie beispielsweise p-Toluolsulfonsäure, bei Temperaturen zwischen 80 und 100 °C, gegebenenfalls unter erhöhtem Druck, umsetzt.

An die so erhaltenen Verbindungen der Formel (I) kann gegebenenfalls anschließend eine Säure addiert werden. In manchen Fällen erweist es sich als vorteilhaft, die Verbindungen der Formel (I) über ihre Salze in reiner Form zu erhalten.

Die substituierten 1,3-Dioxolan- und -Dioxan-Derivate der Formel (II) sind noch nicht bekannt. Sie werden erhalten, indem man Keto-Derivate der Formel

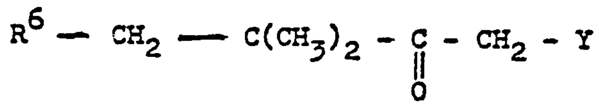

(VI)

in welcher Y und $R^6$ die oben angegebene Bedeutung haben, mit Diolen der Formel (V) gemäß den Bedingungen des Verfahrens (b) umsetzt.

Die Keto-Derivate der Formel (VI) sind bekannt (vergleiche DE-OS 26 35 663, EP-AS 0 042 980, EP-AS 0 054 865). Sie werden z. B. erhalten, indem man die entsprechenden Ketone mit Chlor oder Brom in Gegenwart eines inerten organischen Lösungsmittels, wie beispielsweise Ether, chlorierten oder nicht chlorierten Kohlenwasserstoffen, bei Raumtemperatur umsetzt, oder mit üblichen Chlorierungsmitteln, wie beispielsweise Sulfurylchlorid bei 20 bis 60 °C umsetzt.

Die Alkalisalze von Azolen der Formel (III) sind allgemein bekannt. Sie werden durch Umsetzung von Imidazol bzw. 1,2,4-Triazol mit Natrium- oder Kaliummethylat oder durch Umsetzung von Imidazol bzw. Triazol mit der äquivalenten Menge des entsprechenden Alkalihydrids erhalten. Die Azolmethyl-keto-Derivate der Formel (IV) sind bekannt (vergleiche DE-OS 24 31 407, DE-OS 29 06 061, EP-OS 0 044 993, EP-OS 0 054 865).

Sie werden erhalten, indem man Keto-Derivate der Formel (VI) mit Alkalisalzen von Azolen der Formel (III) gemäß den Bedingungen des Verfahrens (a) umsetzt, oder mit Azolen direkt in üblicher Weise in Gegenwart eines Säurebinders umsetzt.

Die Diole der Formel (V) sind allgemein bekannte Verbindungen der organischen Chemie bzw. werden sie in allgemein bekannter Art und Weise erhalten.

Zur Herstellung von physiologisch verträglichen Säureadditionssalzen der Verbindungen der Formel (I) kommen vorzugsweise folgende Säuren infrage :

Die Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z. B. p-Toluolsulfonsäure und 1,5-Naphthalindisulfonsäure. Die Säureadditions-Salze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Die erfindungsgemäß verwendbaren Verbindungen der Formel (I) ihre Säureadditions-Salze weisen antimikrobielle, insbesondere starke antimykotische Wirkungen auf. Sie besitzen ein sehr breites antimykotisches Wirkungsspektrum, insbesondere gegen Dermatophyten und Sproßpilze sowie bisphasische Pilze, z. B. gegen Candida-Arten, wie Candida albicans, Epidermophyton-Arten, wie Epidermophyton floccosum, Aspergillus-Arten, wie Aspergillus niger und Aspergillus fumigatus, wie Trichophyton-Arten, wie Trichophyton mentagrophytes, Microsporon-Arten, wie Microsporon felineum sowie Penicillium-Arten, wie Penicillium commune. Die Aufzählung dieser Mikroorganismen stellt keinesfalls eine Beschränkung der bekämpfbaren Keime dar, sondern hat nur erläuternden Charakter.

Als Indikationsgebiete in der Humanmedizin können beispielsweise genannt werden :

Dermatomykosen und Systemmykosen durch Trichophyton mentagrophytes und andere Trichophytonarten, Mikrosporonarten, Epidermophyton floccosum, Sproßpilze und biphasische Pilze sowie Schimmelpilze hervorgerufen.

Als Indikationsgebiet in der Tiermedizin können beispielsweise aufgeführt werden : Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoff bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z. B. Tabellen, Dragges, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z. B. 1,2,3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z. B. Stärken, Milchzucker, Rohrzucker, Glukose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumbicarbonat, (e) Lösungsverzögerer, z. B. Paraffin und (f) Resorptionsbeschleuniger, z. B. quartenäre Ammoniumverbindungen, (g) Netzmittel, z. B. Cetylalkohol, Glycerinmonostearat, (h) Adsorptionsmittel, z. B. Kaolin und Bentonit und (i) Gleitmittel, z. B. Talkum, Calcium- und Magnesiumstearat und feste Polyäthylenglykole oder Gemische der unter (a) bis (i) aufgeführten Stoffe.

Den Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien können neben dem oder den Wirkstoffen die üblichen wasserlöslichen oder wasserunlöslichen Trägerstoffe enthalten, z. B. Polyäthylenglykole, Fette, z. B. Kakaofett und höhere Ester (z. B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polyäthylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel z. B. Chlorfluorkohlenwasserstoffe enthalten.

Lösungen und Emulsionen können neben dem oder den Wirkstoffen die üblichen Trägerstoffe der Lösungsmittel, Lösungsvermittler und Emulgatoren, z. B. Wasser, Äthylalkohol, Isopropylalkohol, Äthylcarbonat, Äthylacetat, Benzylalkohol, Benzylenzoat, Propylenglykol, 1,3-Butylenglykol, Dimethylformamid, Oele, insbesondere Baumwollsaatöl, Erdnußöl, Meiskeimöl, Olivenöl, Ricinusöl und Sesaöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z. B. Wasser, Äthylalkohol, Propylenglykol, Suspendiermittel, z. B. äthoxylierte Isostearylalkohole, Polyoxyäthylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmackverbessernde Zusätze, z. B. Pfefferminzöl und Eukalyptusöl und Süßmittel, z. B. Sacharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, in der Human- und Veterinärmedizin zur Verhütung, Besserung und/oder Heilung der oben angeführten Erkrankungen.

Die Wirkstoffe oder die pharmazeutischen Zubereitungen können lokal, oral, parenteral, intraperitoneal und/oder rectal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 10 bis etwa 300, vorzugsweise 50 bis 200 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen und zwar in Abhängigkeit von der Art und dem Körpergewicht des zu behandelnden Objekts der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Herstellungsbeispiele

Beispiel 1

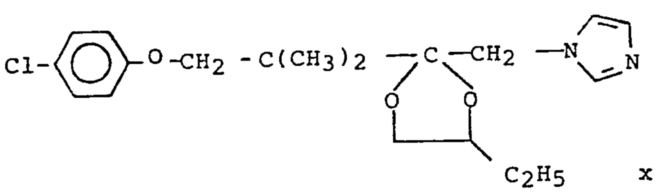

x HCl

Verfahren (a)

25,8 g ($3{,}8 \times 10^{-1}$ Mol) Imidazol werden in 600 ml Dimethylformamid gelöst, 20,5 g ($3{,}8 \times 10^{-1}$ Mol) Natriummethylat, gelöst in 60 ml Methanol, zugetropft und das Methanol abdestilliert. Bei 80 °C werden 74 g ($1{,}9 \times 10^{-1}$ Mol) rohes 2-Brommethyl-2-[β-(4-chlorphenoxy)-α,α-dimethyl]-ethyl-4-ethyl-1,3-dioxolan (Gehalt 62 % GC) zugetropft und weitere 6 Stunden unter Rückfluß erhitzt. Nach Abkühlen wird in 2 l Wasser eingerührt, mit zweimal 500 ml Toluol extrahiert, die vereinigten Toluolphasen dreimal mit je 250 ml Wasser extrahiert und das Lösungsmittel im Wasserstrahlvakuum abdestilliert. Der Rückstand wird in 300 ml Diisopropyläther aufgenommen und mit gesättigter ätherischer Salzsäure versetzt. Der entstehende Niederschlag wird abgesaugt. Man erhält 39,7 g (84 % der Theorie) 2-(Imidazol-1-yl)-methyl-2-[β-(4-chlorphenoxy)-α,α-dimethyl]-ethyl-4-ethyl-1,3-dioxolan-hydrochlorid vom Schmelzpunkt 146-47 °C.

Herstellung des Ausgangsproduktes

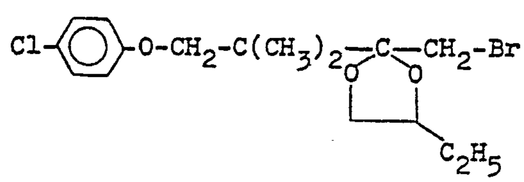

91 g ($3 \times 10^{-1}$ Mol) 1-Brom-3,3-dimethyl-4-(4-chlorphenoxy)-butan-2-on werden in 400 ml Toluol gelöst, 54 g ($6 \times 10^{-1}$ Mol) 1,2-Butandiol sowie 5,2 ($3 \times 10^{-2}$ Mol) p-Toluolsulfonsäure zugegeben und das Reaktionsgemisch anschließend 16 Stunden am Wasserabscheider unter Rückfluß erhitzt. Nach Abkühlen wird die organische Phase mit zweimal 250 ml gesättigter Natriumhydrogencarbonatlösung gewaschen und das Lösungsmittel im Wasserstrahlvakuum abdestilliert. Man erhält 120 g rohes 2-Brommethyl-2[β-(4-chlorphenoxy)-α,α-dimethyl]-ethyl-4-ethyl-1,3-dioxoxolan (GC-Gehalt 62 %), das direkt weiter umgesetzt wird.

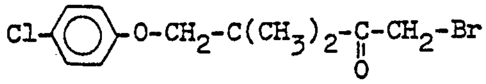

26 g (0,159 Mol) 1-(4-Chlorphenoxy)-2,2-dimethyl-butan-3-on werden in 300 ml Chloroform gelöst und bei 20 °C tropfenweise so mit 25,5 g (0,159 Mol) Brom versetzt, daß laufend Entfärbung eintritt. Nach beendeter Zugabe läßt man 30 Minuten bei Raumtemperatur rühren und engt danach durch Abdestillieren des Lösungsmittels im Vakuum ein. Man erhält 48,5 g (quantitativer Umsatz) 1-Brom-4-(4-chlorphenoxy)-3,3-dimethylbutan-2-on vom Siedepunkt 150-160 °C/0,14 Torr.

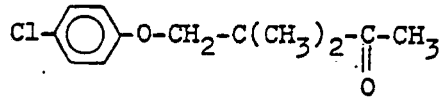

29,7 g (0,55 Mol) Natriummethylat werden in 500 ml Methanol gelöst und unter Rühren mit 70,4 g (0,55 Mol) 4-Chlorphenol versetzt. Nach 10 Minuten Rühren wird das Lösungsmittel unter vermindertem Druck abdestilliert und der Rückstand in 100 ml Glykol aufgenommen. Diese Lösung wird zu einer Lösung von 135 g (0,5 Mol) 2,2-Dimethyl-1-tosyloxy-butan-3-on in 200 ml Glykol gegeben. Man läßt 48 Stunden bei 100 bis 120 °C rühren, kühlt ab und rührt das Reaktionsgemisch in 2 000 ml Wasser ein. Man extrahiert zweimal mit je 250 ml Diethylether, wäscht die vereinigten organischen Phasen dreimal mit je 100 ml Wasser, einmal mit 100 ml 10 %-iger Natronlauge und nochmals mit 100 ml Wasser, trocknet über Natriumsulfat und destilliert. Man erhält 62,9 g (55,7 % der Theorie) 1-(4-Chlorphenoxy)-2,2-dimethyl-butan-3-on vom Siedepunkt 135-140 °C/0,4 Torr.

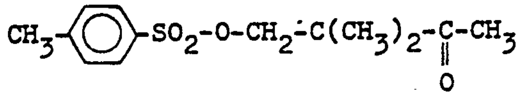

47,6 g (0,25 Mol) 4-Toluolsulfochlorid werden in 100 ml Chloroform gelöst, 35 g (0,3 Mol) 2,2-Dimethyl-1-hydroxy-butan-3-on zugegeben und bei 0 bis 5 °C 40 ml (0,5 Mol) Pyridin zugetropft. Man läßt 15 Stunden bei Raumtemperatur nachrühren, gibt das Reaktionsgemisch auf 200 g Eis und 70 ml konzentrierte Salzsäure, trennt die organische Phase ab, wäscht sie dreimal mit je 200 ml Wasser nach, trocknet über Natriumsulfat und engt ein. Der Rückstand wird in 100 ml Petrolether aufgenommen, wobei das Endprodukt auskristallisiert. Man erhält 46 g (71 % der Theorie) 2,2-Dimethyl-1-tosyloxy-butan-3-on als farblose Kristalle vom Schmelzpunkt 49-52 °C.

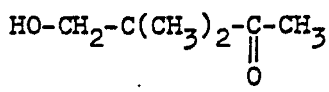

Zu 172 g (2 Mol) Methylisopropylketon in 1 000 ml Methanol werden 66 g (2,2 Mol) Paraformaldehyd und 1 g Kaliumhydroxid in 10 ml Methanol getropft. Man erhitzt 15 Stunden unter Rückfluß und destilliert anschließend das Methanol über eine Kolonne bei 82 °C Innentemperatur ab. Der Rückstand wird im Wasserstrahlvakuum destilliert. Man erhält 152,7 g (68 % der Theorie) 2,2-Dimethyl-1-hydroxy-butan-3-on vom Siedepunkt 80-82 °C/12 Torr.

Beispiel 2

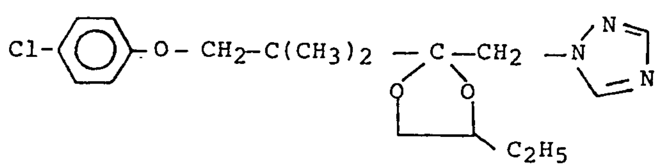

(Verfahren a)

Zu 21,4 g ($3,1 \times 10^{-1}$ Mol) 1,2,4-Triazol in 600 ml Dimethylformamid werden 16,8 g ($3,1 \times 10^{-1}$ Mol) Natriummethylat in 60 ml Methanol getropft und das Methanol abdestilliert. Bei 80 °C werden 60 g ($1,56 \times 10^{-1}$ Mol) 2-Brommethyl-2-[β-(4-chlorphenoxy)-α,α-dimethyl]-ethyl-4-ethyl-dioxolan (Gehalt 62 % GC) zugetropft und das Reaktionsgemisch 15 Stunden unter Rückfluß erhitzt. Die abgekühlte Dimethylformamid-Lösung wird in 2 l Wasser eingerührt und mit zweimal 250 ml Toluol extrahiert. Die Toluolphase wird mit dreimal 250 ml Wasser gewaschen, über Natriumsulfat getrocknet und das Lösungsmittel im Wasserstrahlvakuum abdestilliert. Man erhält 20 g rohes Produkt, das in 200 ml Diethylether aufgenommen und mit 20 ml gesättigter etherischer Salzsäure versetzt wird. Das Lösungsmittel wird abdestilliert und der Rückstand erneut in 200 ml Ether aufgenommen. Man erhält ein Öl, von dem die

etherische Phase abdekantiert wird. Nach Chromatographie an einer Kieselgel-Säule (250 g Kieselgel 60) in Chloroform-Methanol erhält man 7,8 g (17,4 % der Theorie) 2-(1,2,4-Triazol-1-yl)-methyl-2-[β-(4-chlorphenoxy)-α,α-dimethyl]-ethyl-4-ethyl-1,3-dioxolan vom Schmelzpunkt 109 °C.

In entsprechender Weise und gemäß den angegebenen Verfahren werden die folgenden Verbindungen der allgemeinen Formel

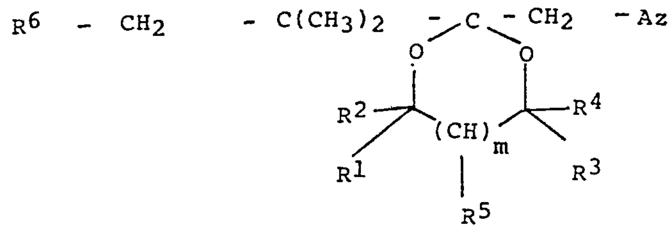 (I)

erhalten :

| Bsp. Nr. | 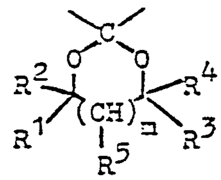 | $R^6$ | Az | Fp. (°C) |
|---|---|---|---|---|
| 3 | 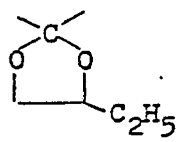 | H | 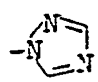 | 220 (x HCl) |
| 4 | 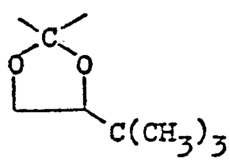 | H | " | 132 (x HCl) |
| 5 | 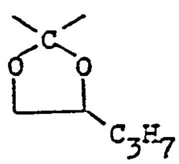 | H | " | 190-92 (x HCl) |
| 6 | 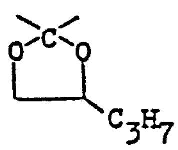 | 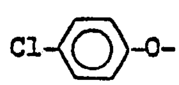 | 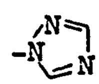 | 145 (x HCl) |
| 7 | 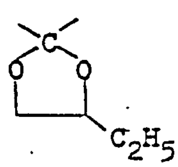 | 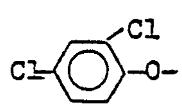 | " | 156 (x HCl) |
| 8 | 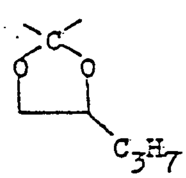 | 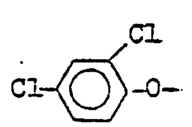 | " | 144 (x HCl) |

(Fortsetzung)

| Bsp. Nr. | 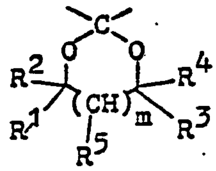 | $R^6$ | Az | Fp.(°C) |
|---|---|---|---|---|
| 9 | 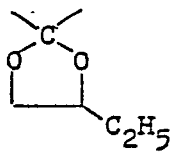 | 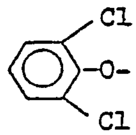 | ″ | 138 (x HCl) |
| 10 | 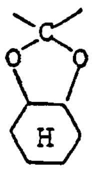 | 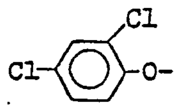 | ″ | 142 (x HCl) |
| 11 | 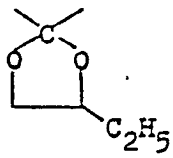 | 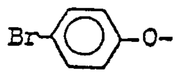 | ″ | 174 (x HCl) |
| 12 | 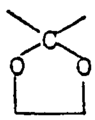 | 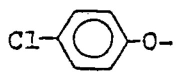 | 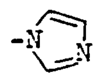 | 169 (x HCl) |
| 13 | 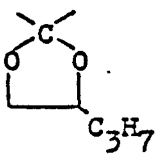 | 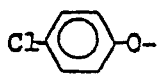 | 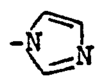 | 153-154 (x HCl) |
| 14 | 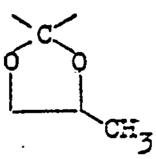 | 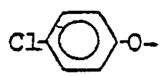 | ″ | 127 (x HCl) |
| 15 | 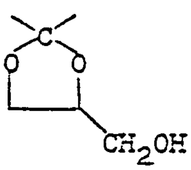 | 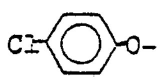 | ″ | 148 (x HCl) |
| 16 | 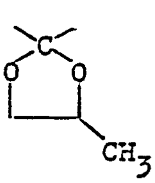 | 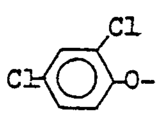 | ″ | 166 (x HCl) |

(Fortsetzung)

| Bsp. Nr. | 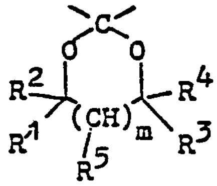 | $R^6$ | Az | Fp.(°C) |
|---|---|---|---|---|
| 17 | 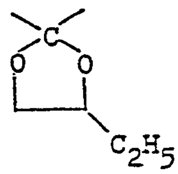 | 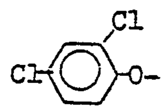 | " | 158 (x HCl) |
| 18 | 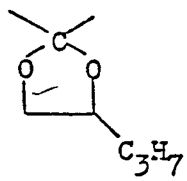 | 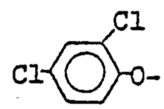 | " | 154 (x HCl) |
| 19 | 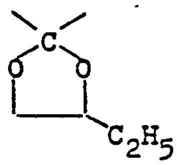 | 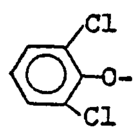 | 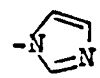 | 169 (x HCl) |
| 20 | 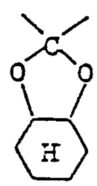 | 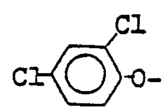 | " | 135 (x HCl) |
| 21 | 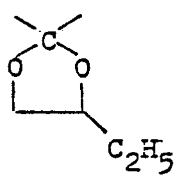 | 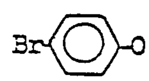 | " | 176 (x HCl) |
| 22 | 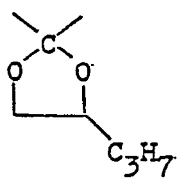 | H | " | 173 (x HCl) |
| 23 | 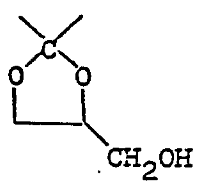 | H | " | 168 (x HCl) |
| 24 | 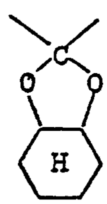 | H | " | 222 (x HCl) |

(Fortsetzung)

| Bsp. Nr. | 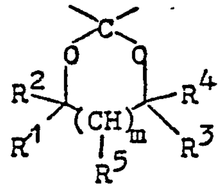 | $R^6$ | Az | Fp.(°C) |
|---|---|---|---|---|
| 25 | 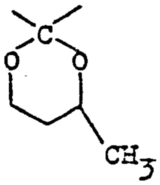 | 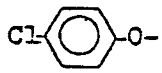 | 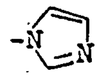 | zähes Öl |
| 26 | 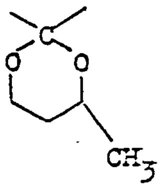 | 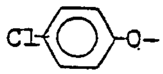 | " | 162 (x HCl) |
| 27 | 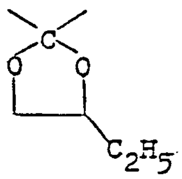 | 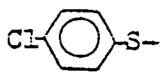 | " | 92 |
| 28 | 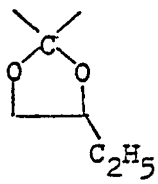 | 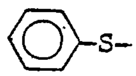 | " | zähes Öl |
| 29 | 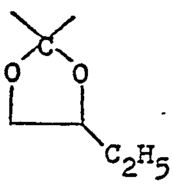 | 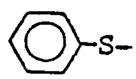 | 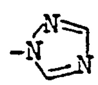 | zähes Öl |
| 30 | 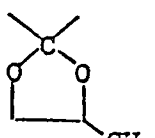 | 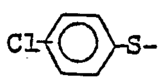 | " | zähes Öl |
| 31 | 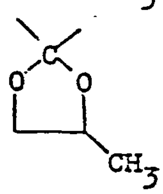 | 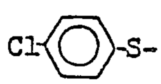 | 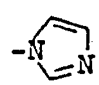 | zähes Öl |
| 32 | 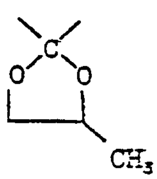 | 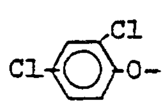 | 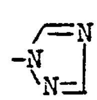 | 152 (xHCl) |

(Fortsetzung)

| Bsp. Nr. | 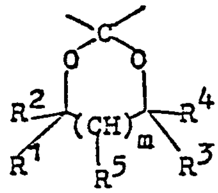 | $R^6$ | Az | Fp. (°C) |
|---|---|---|---|---|
| 33 | 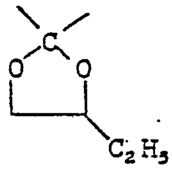 | 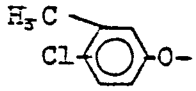 | 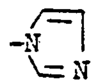 | 170 (xHCl) |
| 34 | 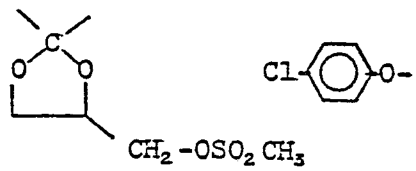 | | 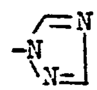 | Kristall-brei |
| 35 | 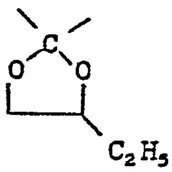 | 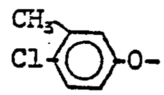 | 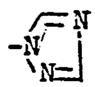 | 167 (xHCl) |
| 36 | 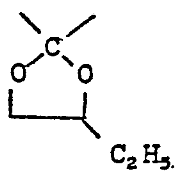 | 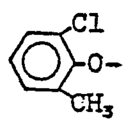 | 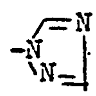 | 129 (xHCl) |
| 37 | 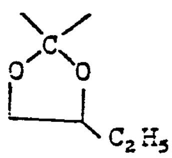 | 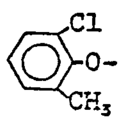 | 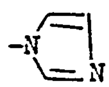 | 184 (xHCl) |
| 38 | 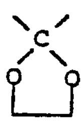 | 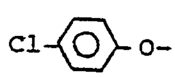 | 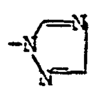 | 180–82 (xHCl) |
| 39 | 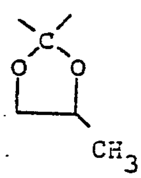 | 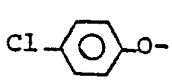 | 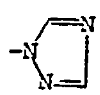 | 179–82 (xHCl) |
| 40 | 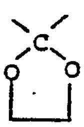 | 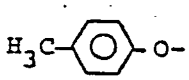 | 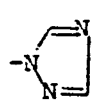 | $n_D^{20}$ = 1,5142 |

(Fortsetzung)

| Bsp. Nr. | 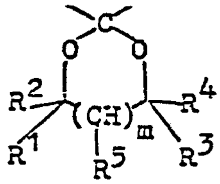 | $R^6$ | Az | Fp. (°C) |
|---|---|---|---|---|
| 41 | 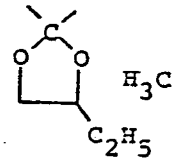 | 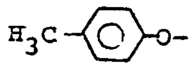 | 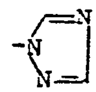 | $n_D^{20}$ = 1,5264 |
| 42 | 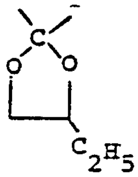 | 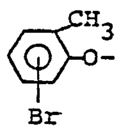 | 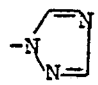 | $n_D^{20}$ = 1,5283 |
| 43 | 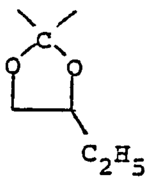 | 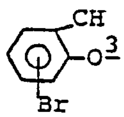 | 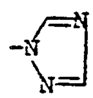 | 137 (x $\frac{1}{2}$ NDS)* |
| 44 | 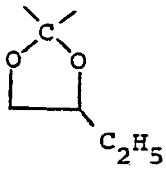 | 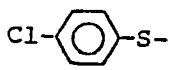 | " | Öl |
| 45 | 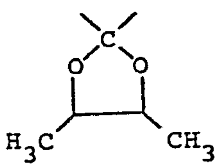 | 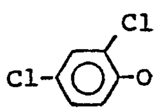 | " | 180 (x HCl) |
| 46 | 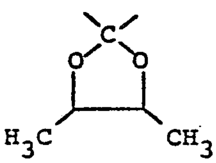 | 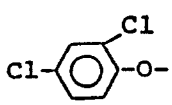 | 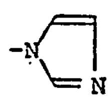 | 166-68°C (x HCl) |
| 47 | 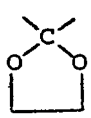 | 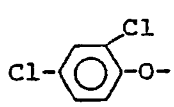 | " | 172°C (x HCl) |
| 48 | 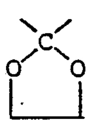 | 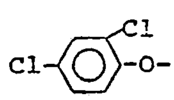 | 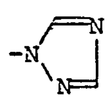 | 157-59°C (x HCl) |

NDS+ = 1,5-Naphthalindisulfonsäure

(Fortsetzung)

| Bsp. Nr. | 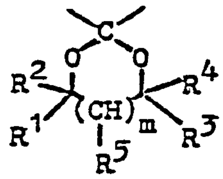 | $R^6$ | Az | Fp. (°C) |
|---|---|---|---|---|
| 49 | 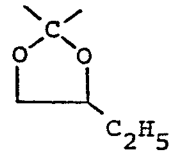 | 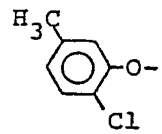 | 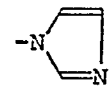 | 147-49°C (x HCl) |
| 50 | 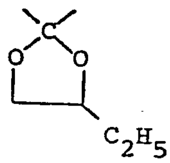 | 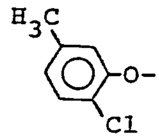 | 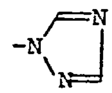 | 90°C (x HCl) |
| 51 | 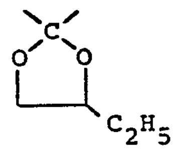 | 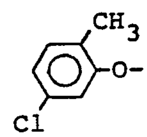 | 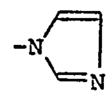 | 160°C (x HCl) |
| 52 | 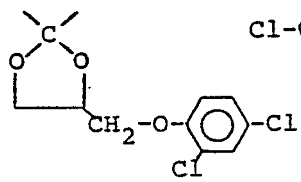 | 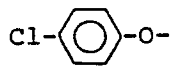 | " | 170°C |
| 53 | 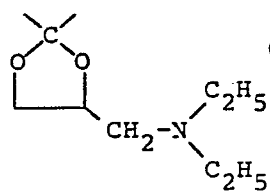 | 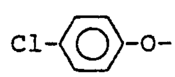 | " | halb. Krist. |
| 54 | 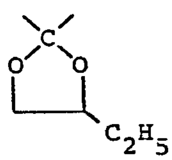 | 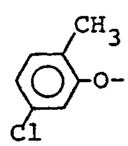 | 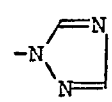 | 150°C (x HCl) |
| 55 | 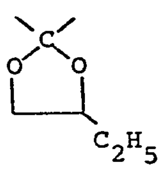 | 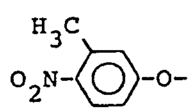 | " | 61°C |
| 56 | 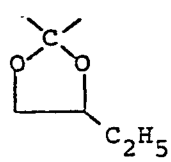 | 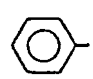 | 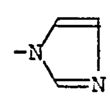 | 102-4°C |

(Fortsetzung)

| Bsp. Nr. | 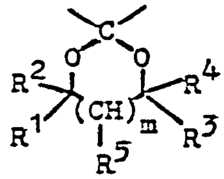 | $R^6$ | Az | Fp.(°C) |
|---|---|---|---|---|
| 57 | 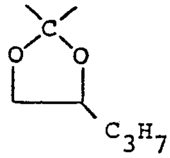 | 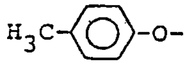 | 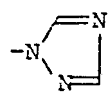 | $n_D^{20}$ = 1,5419 |
| 58 | 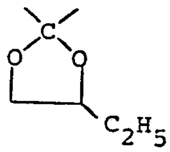 | 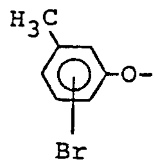 | " | $n_D^{20}$ = 1,5080 |
| 59 | 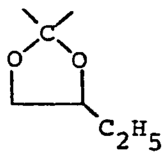 | 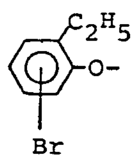 | " | $n_D^{20}$ = 1,5252 |
| 60 | 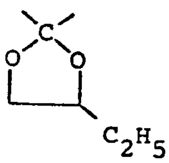 | 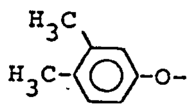 | " | $n_D^{20}$ = 1,5368 |
| 61 | 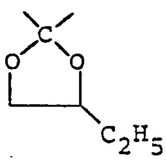 | 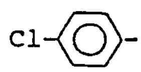 | 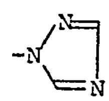 | 138°C |
| 62 | 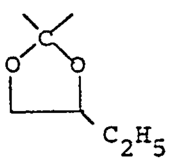 | 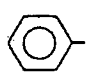 | " | 97°C |
| 63 | 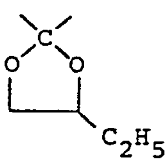 | 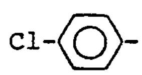 | 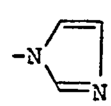 | 137°C |

(Fortsetzung)

| Bsp. Nr. | 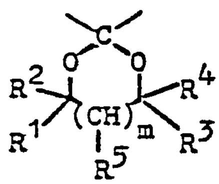 | $R^6$ | Az | Fp.(°C) |
|---|---|---|---|---|
| 64 | 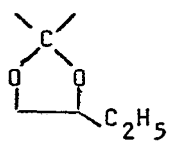 | 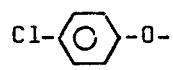 | 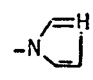 | zähes Öl |
| 65 | 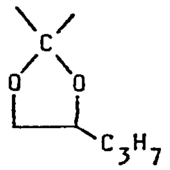 | 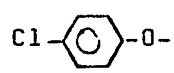 | 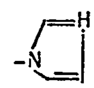 | 153-54°C (xHCl). |

Verwendungsbeispiele :

In den nachfolgenden Beispielen werden die nachstehend angegebenen Verbindungen als Vergleichssubstanzen eingesetzt :

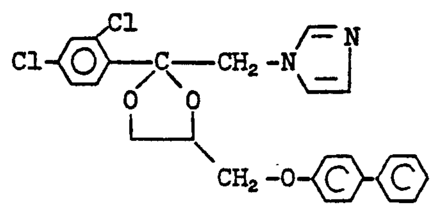

(A)

4-(4-Biphenylyloxymethyl)-2-(2,4-dichlorphenyl)-2-(imidazol-1-yl-methyl)-1,3-dioxolan

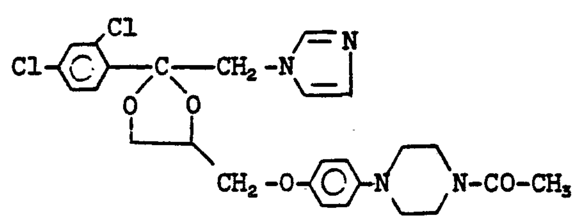

(B)

4-[4-(4'-Acetylpiperazin-1-yl)-phenoxymethyl]-2-(2,4-dichlorphenyl)-2-(imidazol-1-yl-methyl)-1,3-dioxolan

Beispiel A

Antimykotische in vitro-Wirksamkeit

Versuchsbeschreibung :

Die in vitro-Prüfungen wurden im Reihenverdünnungstest mit Keiminokula von durchschnittlich $5 \times 10^4$ Keimen/ml Substrat durchgeführt. Als Nährmedium dienten

a) für Dermatophyten und Schimmelpilze : Sabourand's milieu d'épreuve

b) für Hefen : Fleischextrakt-Traubenzucker-Bouillon.

Die Bebrütungstemperatur betrug 20 °C, die Bebrütungsdauer lag bei 24 bis 96 Stunden bei Hefen und 96 Stunden bei Dermatophyten und Schimmelpilzen.

In diesem Test zeigen insbesondere die Verbindungen der Beispiele 1,2,12,13,14 und 25 eine bessere antimykotische Wirkung als die aus dem Stand der Technik bekannten Verbindungen (A) und (B).

**Ansprüche**

1. Antimykotische Mittel zur Behandlung von Menschen und Tieren gekennzeichnet durch einen Gehalt an mindestens einem 2-Azolylmethyl-1,3-dioxolan- oder -dioxan der allgemeinen Formel

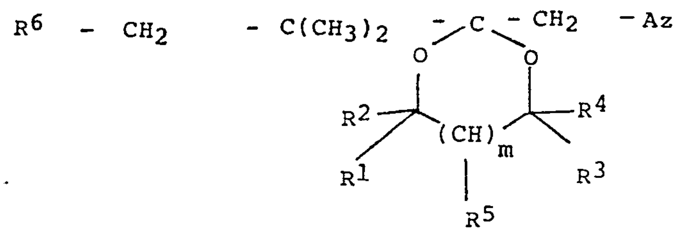

(I)

in welcher

Az für Imidazol-1-yl oder 1,2,4-Triazol-1-yl steht ;

$R^1$ für Wasserstoff oder geradkettiges und verzweigtes Alkyl mit 1 bis 4 Kohlenstoffatomen steht ;

$R^2$ für Wasserstoff oder geradkettiges und verzweigtes Alkyl mit 1 bis 4 Kohlenstoffatomen steht ;

$R^3$ für Wasserstoff oder geradkettiges und verzweigtes Alkyl mit 1 bis 4 Kohlenstoffatomen steht ;

$R^4$ für Wasserstoff, geradkettiges oder verzweigtes Alkyl mit 1 bis 4 Kohelnstoffatomen, das gegebenenfalls substituiert sein kann durch : Hydroxy, Alkoxy mit 1 bis 4 Kohlenstoffatomen, Alkylcarbonyloxy mit 1 bis 2 Kohlenstoffatomen im Alkylrest, Dialkyl amino und Dialkylaminocarbonyl mit jeweils 1 bis 2 Kohlenstoffatomen in jedem Alkylteil, gegebenenfalls substituiertes Phenoxy und gegebenenfalls substituiertes Phenylalkoxy mit 1 bis 4 Kohlenstoffatomen im Alkylteil, gegebenenfalls substituiertes Phenylcarbonyloxy und gegebenenfalls substituiertes Phenylalkylcarbonyloxy mit 1 bis 4 Kohlenstoffatomen im Alkylteil, Alkylsulfonyloxy mit 1 bis 4 Kohlenstoffatomen und gegebenenfalls substituiertes Phenylsulfonyloxy, wobei jeweils als Phenylsubstituenten genannt seien : Halogen, Alkyl mit 1 bis 4 Kohlenstoffatomen, Alkoxy und Alkylthio mit jeweils 1 bis 2 Kohlenstoffatomen, Halogenalkyl, Halogenalkoxy und Halogenalkylthio mit jeweils 1 bis 2 Kohlenstoff- und 1 bis 5 gleichen oder verschiedenen Halogenatomen, wie insbesondere Fluor- und Chloratoren, Dimethylamino, Acetylamino, Acetyl-methylamino, gegebenenfalls durch Methyl oder Acetyl substituiertes Piperazin ; sowie schließlich für gegebenenfalls substituiertes Phenyl und Phenylalkyl mit 1 bis 4 Kohlenstoffatomen im Alkylteil steht, wobei als Phenylsubstituenten die oben genannten infrage kommen ;

$R^1$ und $R^3$ außerdem gemeinsam für eine gegebenenfalls durch Alkyl mit 1 bis 4 Kohlenstoffatomen substituierte Tetra- oder Pentamethylenbrücke stehen ;

$R^5$ für Wasserstoff oder geradkettiges und verzweigtes Alkyl mit 1 bis 4 Kohlenstoffe steht ;

$R^6$ für Wasserstoff, Halogen, Cyano, geradkettiges oder verzweigtes Alkyl mit 1 bis 4 Kohlenstoffatomen, gegebenenfalls substituiertes Phenyl, wobei als Phenylsubstituenten die bei $R^4$ bereits genannten Phenylsubstituenten infrage kommen, sowie für die Gruppierunen $—X—R^7$, $—COOR^8$ und $—CONHR^9$ steht,

$R^7$ für geradkettiges oder verzweigtes Alkyl mit 1 bis 4 Kohlenstoffatomen, Halogenalkyl mit 1 bis 2 Kohlenstoff- und 1 bis 5 gleichen oder verschiedenen Halogenatomen, wie insbesondere Fluor- und Chloratomen, Cyano, sowie für gegebenenfalls substituiertes Phenyl und Phenylalkyl mit 1 bis 4 Kohlenstoffatomen im Alkylteil steht, wobei jeweils als Phenylsubstituenten die bei $R^4$ bereits genannten Phenylsubstituenten infrage kommen ;

$R^8$ für geradkettiges oder verzweigtes Alkyl mit 1 bis 4 Kohlenstoffatomen steht ;

$R^9$ für geradkettiges oder verzweigtes Alkyl mit 1 bis 4 Kohlenstoffatomen sowie für gegebenenfalls substituiertes Phenyl steht, wobei als Phenylsubstituenten die bei $R^4$ bereits genannten Phenylsubstituenten infrage kommen ;

X für Sauerstoff, Schwefel, die SO- oder $SO_2$-Gruppe steht ; und

m für 0 oder 1 steht,

wobei die Gruppierung $R^6—CH_2—$ nicht für Methyl stehen darf, wenn m = 0, Az = 1,2,4-Triazol-1-yl und $R^4$ gegebenenfalls substituiertes Phenoxyalkyl bedeuten, sowie deren Säureadditionssalze.

2. Antimykotisches Mittel zur Behandlung von Menschen und Tieren gekennzeichnet durch einen Gehalt an mindestens einem 2-Azolylmethyl-1,3-dioxolan- oder -dioxan-Derivate der allgemeinen Formel

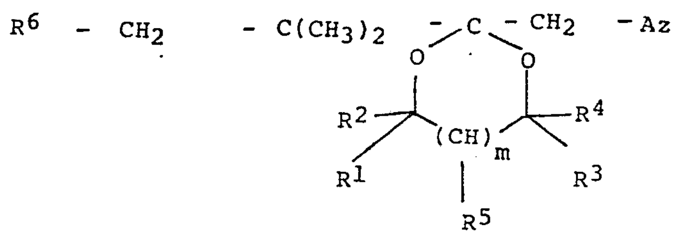

(I)

in welcher

$R^1$, $R^2$ und $R^4$ die in Anspruch 1 angegebene Bedeutung haben ;

$R^4$ für Wasserstoff oder geradkettiges und verzweigtes Alkyl mit 1 bis 4 Kohlenstoffatomen steht, das gegebenenfalls substituiert sein kann durch : Hydroxy, Methoxy, Ethoxy, Dimethylamino, Dimethylaminocarbonyl, gegebenenfalls substituiertes Phenoxy und Benzyloxy, Methylcarbonyloxy, Ethylcarbonyloxy, gegebenenfalls substituiertes Phenylcarbonyloxy und Benzylcarbonyloxy, Methylsulfonyloxy, Ethylsulfonyloxy sowie gegebenenfalls substituiertes Phenylsulfonyloxy, wobei jeweils als Phenylsubstituenten genannt seien : Fluor, Chlor, Methyl, Ethyl, Methoxy, Methylthio, Trifluormethyl und Trifluormethylthio, Dimethylamino, Acetylamino, Acetyl-methylamino, 4-Acetylpiperazin-1-yl, sowie auch für gegebenenfalls substituiertes Phenyl und Benzyl steht, wobei als Phenylsubstituenten die obengenannten infrage kommen ;

$R^1$ und $R^3$ außerdem gemeinsam für Tetramethylenbrücke stehen ;

$R^5$ für Wasserstoff, Methyl oder Ethyl steht ;

$R^6$ für Wasserstoff, Fluor, Chlor, Brom, Methyl, Ethyl, Propyl, gegebenenfalls substituiertes Phenyl, wobei als Substituenten die bei $R^4$ bereits genannten Phenylsubstituenten infrage kommen, sowie für die Gruppierungen $—X—R^7$, $—COOR^8$ und $—CONHR^9$ steht,

$R^7$ für Methyl, Ethyl, Trifluormethyl, Cyano, sowie gegebenenfalls substituiertes Phenyl und Benzyl steht, wobei jeweils als Phenylsubstituenten die bei $R^4$ bereits genannten Phenylsubstituenten infrage kommen ;

$R^8$ für Methyl oder Ethyl steht ;

$R^9$ für Methyl, Ethyl sowie gegebenenfalls substituiertes Phenyl steht, wobei als Phenylsubstituenten die bei $R^4$ bereits genannten Phenylsubstituenten infrage kommen ; und

Az, X und m die in Anspruch 1 angegebene Bedeutung haben,

wobei die Gruppierung $R^6—CH_2—$ nicht für Methyl stehen darf, wenn m = 0, Az = 1,2,4-Triazol-1-yl und $R^4$ gegebenenfalls substituiertes Phenoxyalkyl bedeuten, sowie deren Säureadditionssalze.

3. Antimykotisches Mittel zur Behandlung von Menschen und Tieren, gekennzeichnet durch einen Gehalt an der Verbindung der Formel

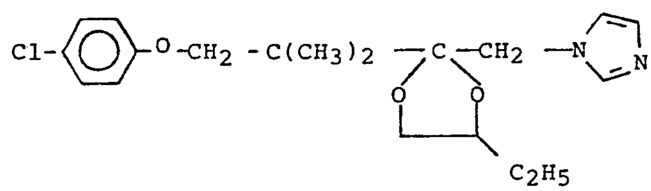

4. Antimykotisches Mittel zur Behandlung von Menschen und Tieren, gekennzeichnet durch einen Gehalt an der Verbindung der Formel

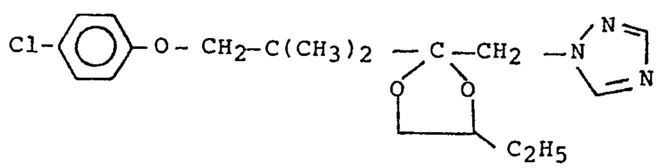

5. Antimykotisches Mittel zur Behandlung von Menschen und Tieren, gekennzeichnet durch einen Gehalt an der Verbindung der Formel

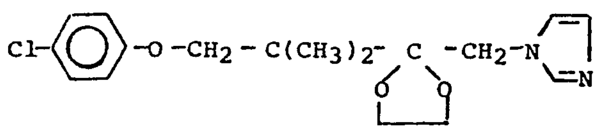

6. Antimykotisches Mittel zur Behandlung an Menschen und Tieren, gekennzeichnet durch einen Gehalt an der Verbindung der Formel

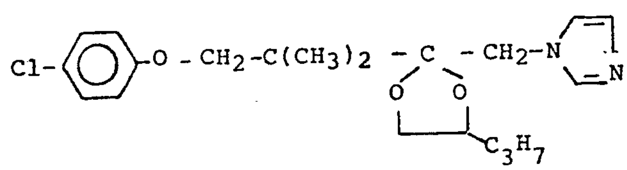

7. Antimykotisches Mittel zur Behandlung von Menschen und Tieren, gekennzeichnet durch einen Gehalt an der Verbindung der Formel

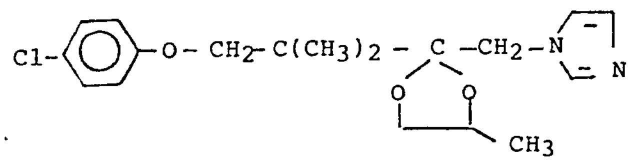

8. Verfahren zur Herstellung eines antimykotischen Mittels, dadurch gekennzeichnet, daß man 2-Azolylmethyl-1,3-dioxolan- oder -dioxan-Derivate gemäß der Formel in Anspruch 1 mit inerten, nichttoxischen, pharmazeutisch geeigneten Trägerstoffen vermischt.

**Claims**

1. Antimycotic agents for treating humans and animals, characterised in that it contains at least one 2-azolyl-methyl-1,3-dioxolane or -dioxane of the general formula

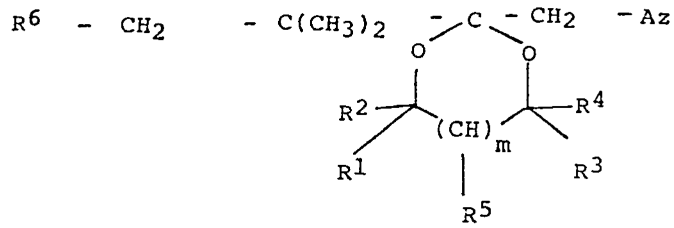

(I)

in which

Az represents imidazol-1-yl or 1,2,4-triazol-1-yl ;

$R^1$ represents hydrogen or straight-chain and branched alkyl having 1 to 4 carbon atoms ;

$R^2$ represents hydrogen or straight-chain and branched alkyl having 1 to 4 carbon atoms,

$R^3$ represents hydrogen or straight-chain and branched alkyl having 1 to 4 carbon atoms ;

$R^4$ represents hydrogen, straight-chain or branched alkyl which has 1 to 4 carbon atoms and which can be optionally substituted by hydroxyl, alkoxy having 1 to 4 carbon atoms, alkylcarboxy having 1 or 2 carbon atoms in the alkyl radical, dialkylamino and dialkylaminocarbonyl, each having 1 or 2 carbon atoms in each alkyl part, optionally substituted phenoxy and optionally substituted phenylalkoxy having 1 to 4 carbon atoms in the alkyl part, optionally substituted phenylcarboxy and optionally substituted phenylalkylcarboxy having 1 to 4 carbon atoms in the alkyl part, alkylsulphonyloxy having 1 to 4 carbon atoms and optionally substituted phenylsulphonyloxy, the following being mentioned as phenyl substituents in each case : halogen, alkyl having 1 to 4 carbon atoms, alkoxy and alkylthio, each having 1 or 2 carbon atoms, halogenoalkyl, halogenoalkoxy and halogenoalkylthio, each having 1 or 2 carbon atoms and 1 to 5 identical or different halogen atoms, such as, in particular, fluorine atoms and chlorine atoms, dimethylamino, acetylamino, acetylmethylamino, and piperazine which is optionally substituted by methyl or acetyl ; and $R^4$ finally represents optionally substituted phenyl and phenylalkyl having 1 to 4 carbon atoms in the alkyl part, the abovementioned substituents being suitable phenyl substituents ;

$R^1$ and $R^3$ additionally together represent a tetra- or pentamethylene bridge which is optionally substituted by alkyl having 1 to 4 carbon atoms ;

$R^5$ represents hydrogen or straight-chain and branched alkyl having 1 to 4 carbon atoms ;

$R^6$ represents hydrogen, halogen, cyano, straight-chain or branched alkyl having 1 to 4 carbon atoms, optionally substituted phenyl, the phenyl substituents already mentioned in the case of $R^4$ being suitable phenyl substituents, and the groupings $—X—R^7$, $—COOR^8$ and $—CONHR^9$ ;

$R^7$ represents straight-chain or branched alkyl having 1 to 4 carbon atoms, halogenoalkyl having 1 or 2 carbon atoms and 1 to 5 identical or different halogen atoms, such as, in particular, fluorine atoms and chlorine atoms, cyano and optionally substituted phenyl and phenylalkyl having 1 to 4 carbon atoms in the alkyl part, the phenyl substituents already mentioned in the case of $R^4$ being suitable phenyl substituents in each case ;

$R^8$ represents straight-chain or branched alkyl having 1 to 4 carbon atoms ;

$R^9$ represents straight-chain or branched alkyl having 1 to 4 carbon atoms, and optionally substituted phenyl, the phenyl substituents already mentioned in the case of $R^4$ being suitable phenyl substituents ;

X represents oxygen, sulphur or the SO- or $SO_2$-group ; and

m represents 0 or 1,

and the grouping $R^6—CH_2—$ cannot represent methyl if m = 0, Az = 1,2,4-triazol-1-yl and $R^4$ denotes optionally substituted phenoxyalkyl, and their acid addition salts.

2. An antimycotic agent for treating humans and animals, characterised in that it contains at least one 2-azolyl-methyl-1,3-dioxolane or -dioxane derivative of the general formula

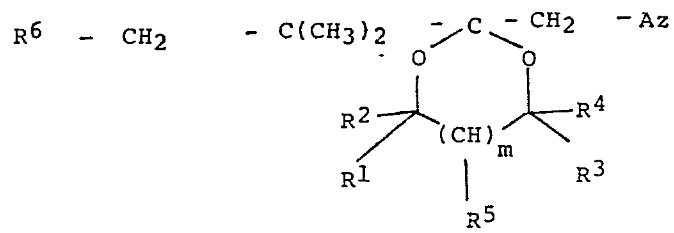

(I)

in which

$R^1$, $R^2$ and $R^3$ have the meaning given in Claim 1 ;

$R^4$ represents hydrogen, or straight-chain and branched alkyl which has 1 to 4 carbon atoms and which can optionally be substituted by hydroxyl, methoxy, ethoxy, dimethylamino, dimethylaminocarbonyl, optionally substituted phenoxy and benzyloxy, methylcarboxy, ethylcarboxy, optionally substituted phenylcarboxy and benzylcarboxy, methylsulphonyloxy, ethylsulphonyloxy and optionally substituted phenylsulphonyloxy, the following being mentioned as phenyl substituents in each case : fluorine, chlorine, methyl, ethyl, methoxy, methylthio, trifluoromethyl and trifluoromethylthio, dimethylamino, acetylamino, acetyl-methyl-amino and 4-acetyl-piperazin-1-yl, and $R^4$ also represents optionally substituted phenyl and benzyl, the abovementioned substituents being suitable phenyl substituents ;

$R^1$ and $R^3$ additionally together represent a tetramethylene bridge ;

$R^5$ represents hydrogen, methyl or ethyl ;

$R^6$ represents hydrogen, fluorine, chlorine, bromine, methyl, ethyl, propyl and optionally substituted phenyl, the phenyl substituents already mentioned in the case of $R^4$ being suitable substituents, and the groupings $-X-R^7$, $-COOR^8$ and $-CONHR^9$ :

$R^7$ represents methyl, ethyl, trifluoromethyl, cyano, and optionally substituted phenyl and benzyl, the phenyl substituents already mentioned in the case of $R^4$ being suitable phenyl substituents in each case ;

$R^8$ represents methyl or ethyl ;

$R^9$ represents methyl, ethyl and optionally substituted phenyl, the phenyl substituents already mentioned in the case of $R^4$ being suitable phenyl substituents ; and

Az, X and m have the meaning given in Claim 1, and the grouping $R^6-CH_2-$ cannot represent methyl if m = 0, Az = 1,2,4-triazol-1-yl and $R^4$ denotes optionally substituted phenoxyalkyl, and their acid addition salts.

3. An antimycotic agent for treating humans and animals, characterised in that it contains the compound of the formula

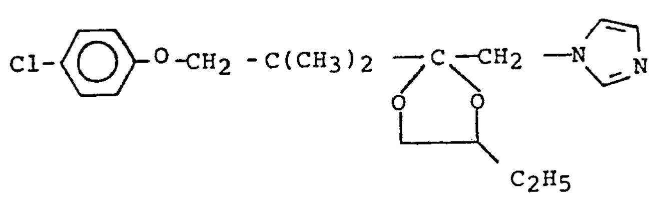

4. An antimycotic agent for treating humans and animals, characterised in that it contains the compound of the formula

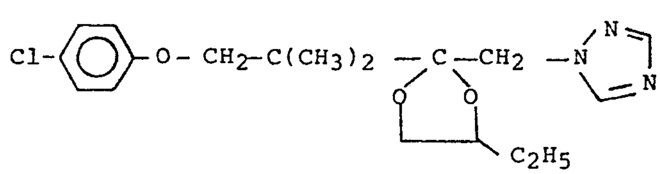

5. An antimycotic agent for treating humans and animals, characterised in that it contains the compound of the formula

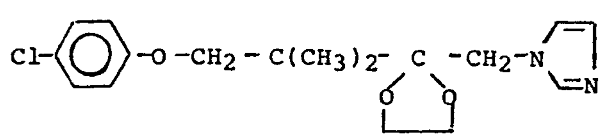

6. An antimycotic agent for treating humans and animals, characterised in that it contains the compound of the formula

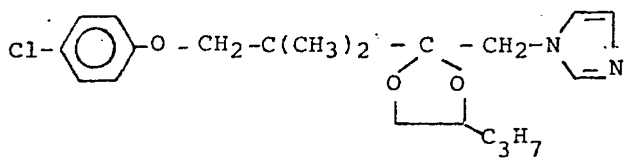

7. An antimycotic agent for treating humans and animals, characterised in that it contains the compound of the formula

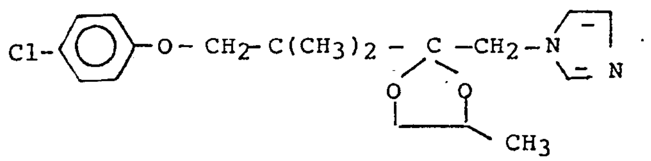

8. Process for the preparation of an antimycotic agent, characterised in that 2-azolylmethyl-1,3-dioxolane or -dioxane derivatives according to the formula in Claim 1 are mixed with inert, non-toxic, pharmaceutically suitable carriers.

**Revendications**

1. Agents antimycotiques pour le traitement des hommes et des animaux, caractérisés par une teneur en au moins un 2-azolylméthyl-1,3-dioxolane ou -dioxane, de formule générale

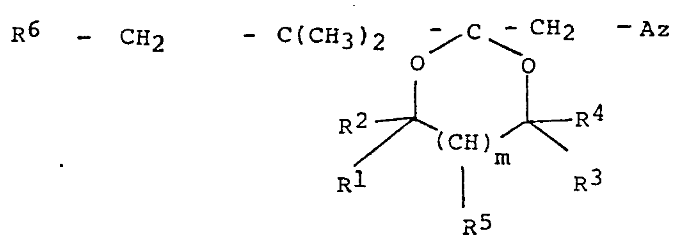

(I)

dans laquelle

Az représente un imidazol-1-yle ou 1,2,4-triazol-1-yle,

$R^1$ de l'hydrogène ou un alcoyle à chaîne droite et ramifiée ayant 1 à 4 atomes de carbone,

$R^2$ de l'hydrogène ou un alcoyle à chaîne droite et ramifiée ayant 1 à 4 atomes de carbone,

$R^3$ de l'hydrogène ou un alcoyle à chaîne droite et ramifiée ayant 1 à 4 atomes de carbone,

$R^4$ de l'hydrogène, un alcoyle à chaîne droite ou ramifiée ayant 1 à 4 atomes de carbone, qui peut éventuellement être substitué par : hydroxy, alcoxy ayant 1 à 4 atomes de carbone, alcoylcarbonyloxy ayant 1 à 2 atomes de carbone dans le radical alcoyle, dialcoylamino et dialcoylaminocarbonyle ayant chaque fois 1 à 2 atomes de carbone dans chaque portion alcoyle, phénoxy éventuellement substitué et phénylalcoxy éventuellement substitué ayant 1 à 4 atomes de carbone dans la portion alcoyle, phénylcarbonyloxy éventuellement substitué et phénylalcoylcarbonyloxy éventuellement substitué ayant 1 à 4 atomes de carbone dans la portion alcoyle, alcoylsulfonyloxy ayant 1 à 4 atomes de carbone et phénylsulfonyloxy éventuellement substitué, en citant chaque fois ici en tant que substituants du phényle : halogène, alcoyle ayant 1 à 4 atomes de carbone, alcoxy et alcoylthio ayant chaque fois 1 à 2 atomes de carbone, halogénoalcoyle, halogénoalcoxy et halogénoalcoylthio ayant chaque fois 1 à 2 atomes de carbone et 1 à 5 atomes d'halogène identiques ou différents, comme en particulier des atomes de fluor et de chlore, diméthylamino, acétylamino, acétylméthylamino, pipérazine éventuellement substituée par un méthyle ou acétyle ; de même que finalement un phényle et phénylalcoyle éventuellement substitués ayant 1 à 4 atomes de carbone dans la portion alcoyle, en envisageant comme substituants du phényle ceux qui ont été cités plus haut,

$R^1$ et $R^3$ représentent en outre ensemble un pont tétra- ou pentaméthylène éventuellement substitué par un alcoyle ayant 1 à 4 atomes de carbone ;

$R^5$ de l'hydrogène ou un alcoyle à chaîne droite et ramifiée ayant 1 à 4 atomes de carbone,

$R^6$ de l'hydrogène, halogène, cyano, alcoyle à chaîne droite ou ramifiée ayant 1 à 4 atomes de carbone, phényle éventuellement substitué, en envisageant comme substituants du phényle les substituants du phényle cités déjà pour $R^4$, de même que les groupements —X—$R^7$, —$COOR^8$ et —$CONHR^9$,

$R^7$ un alcoyle à chaîne droite ou ramifiée ayant 1 à 4 atomes de carbone, halogénoalcoyle ayant 1 à 2 atomes de carbone et 1 à 5 atomes d'halogène identiques ou différents, comme par exemple des atomes de fluor et de chlore, cyano, de même qu'un phényle et phénylalcoyle éventuellement substitués avec 1 à

4 atomes de carbone dans la portion alcoyle, en envisageant chaque fois comme substituants du phényle les substituants du phényle déjà cités pour $R^4$,

$R^8$ un alcoyle à chaîne droite ou ramifiée ayant 1 à 4 atomes de carbone,

$R^9$ un alcoyle à chaîne droite ou ramifiée ayant 1 à 4 atomes de carbone de même qu'un phényle éventuellement substitué, en envisageant comme substituants du phényle les substituants du phényle déjà cités pour $R^4$,

X de l'oxygène, du soufre, le groupe SO ou $SO_2$ et

m 0 ou 1,

le groupement $R^6—CH_2—$ ne pouvant pas être un méthyle lorsque m = 0, Az = 1,2,4-triazol-1-yle et $R^4$ un phénoxyalcoyle éventuellement substitué, ainsi que leurs sels d'addition d'acides.

2. Agents antimycotiques pour le traitement des hommes et des animaux, caractérisés par une teneur en au moins un dérivé de 2-azolylméthyl-1,3-dioxolane ou -dioxane, de formule générale

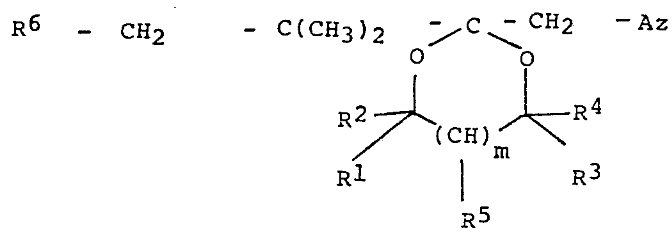

(I)

dans laquelle

$R^1$, $R^2$ et $R^4$ ont la signification indiquée à la revendication 1,

$R^4$ de l'hydrogène ou un alcoyle à chaîne droite et ramifiée ayant 1 à 4 atomes de carbone, qui éventuellement peut être substitué par : hydroxy, méthoxy, éthoxy, diméthylamino, diméthylaminocarbonyle, phénoxy et benzyloxy éventuellement substitués, méthylcarbonyloxy, éthylcarbonyloxy, phénylcarbonyloxy et benzylcarbonyloxy éventuellement substitués, méthylsulfonyloxy, éthylsulfonyloxy de même que phénylsulfonyloxy éventuellement substitué, en mentionnant chaque fois comme substituants du phényle : fluor, chlore, méthyle, éthyle, méthoxy, méthylthio, trifluorométhyle et trifluorométhylthio, diméthylamino, acétylamino, acétylméthylamino, 4-acétyl-pipérazine-1-yle, de même aussi qu'un phényle et benzyle éventuellement substitués, en envisageant comme substituants du phényle ceux qui ont été cités plus haut,

$R^1$ et $R^3$ représentent en outre un pont tétraméthylène,

$R^5$ de l'hydrogène, méthyle ou éthyle,

$R^6$ de l'hydrogène, fluor, chlore, brome, méthyle, éthyle, propyle, phényle éventuellement substitué, en envisageant comme substituants les substituants du phényle déjà cités pour $R^4$, de même que les groupements $—X—R^7$, $—COOR^8$ et $—CONHR^9$,

$R^7$ méthyle, éthyle, trifluorométhyle, cyano, de même que phényle et benzyle éventuellement substitués, en envisageant chaque fois comme substituants du phényle les substituants du phényle déjà cités pour $R^4$,

$R^8$ méthyle ou éthyle,

$R^9$ méthyle, éthyle, de même que phényle éventuellement substitué, en envisageant comme substituants du phényle les substituants du phényle déjà cités pour $R^4$,

Az, X et m ont la signification indiquée à la revendication 1,

le groupement $R^6—CH_2—$ ne pouvant pas être un méthyle lorsque m = 0, Az = 1,2,4-triazol-1-yle et $R^4$ un phénoxyalcoyle éventuellement substitué, ainsi que leurs sels d'addition d'acides.

3. Agent antimycotique pour le traitement des hommes et des animaux, caractérisé par une teneur en composé de formule

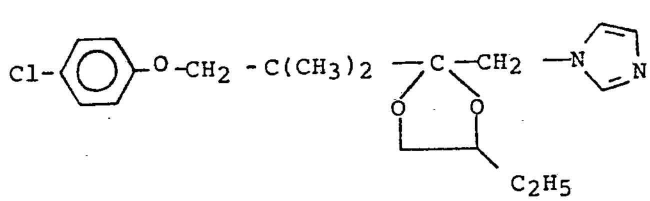

4. Agent antimycotique pour le traitement des hommes et des animaux, caractérisé par une teneur en composé de formule

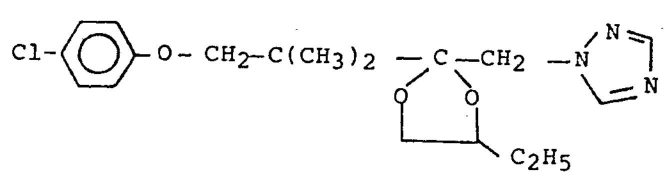

5. Agent antimycotique pour le traitement des hommes et des animaux, caractérisé par une teneur en composé de formule

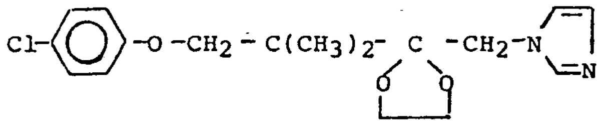

6. Agent antimycotique pour le traitement des hommes et des animaux, caractérisé par une teneur en composé de formule

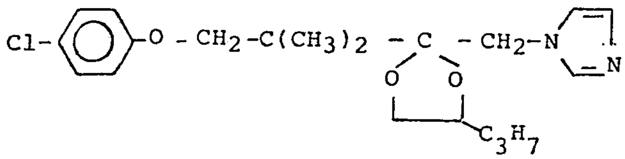

7. Agent antimycotique pour le traitement des hommes et des animaux, caractérisé par une teneur en composé de formule

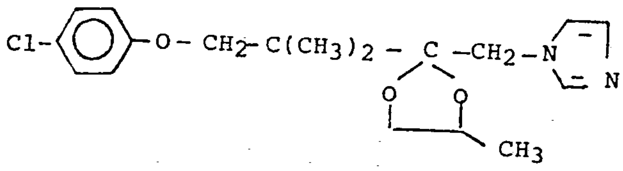

8. Procédé de préparation d'un agent antimycotique, caractérisé en ce qu'on mélange des dérivés de 2-azolylméthyl-1,3-dioxolane ou -dioxane selon la formule donnée à la revendication 1 avec des matières de support inertes, non toxiques et pharmaceutiquement appropriées.